## Europäisches Patentamt
## European Patent Office
## Office européen des brevets

(19)

(11) Publication number: **0 005 291**
**B1**

(12)

# EUROPEAN PATENT SPECIFICATION

(45) Date of publication of patent specification: **23.12.81**

(51) Int. Cl.³: **C 07 C 131/04**

(21) Application number: **79200193.5**

(22) Date of filing: **17.04.79**

(54) Process for the recovery of cyclohexanone oxime.

(30) Priority: **20.04.78 NL 7804195**

(43) Date of publication of application:
**14.11.79 Bulletin 79/23**

(45) Publication of the grant of the European patent:
**23.12.81 Bulletin 81/51**

(84) Designated Contracting States:
**BE DE FR GB IT NL**

(56) References cited:
**GB - A - 1 303 739**

(73) Proprietor: **STAMICARBON B.V.**
**Postbus 10**
**NL-6160 MC Geleen (NL)**

(72) Inventor: **Rulkens, Peter Franciscus Maria**
**Kerkhofweg 23**
**NL-6142 BP Limbricht (NL)**
Inventor: **Wassen, Willem Joseph**
**Moutheuvellaan 4a**
**NL-6365 AZ Schinnen (NL)**

(74) Representative: **Pinckaers, August René et al,**
**OCTROOIBUREAU DSM Postbus 9**
**NL-6160 MA Geleen (NL)**

Courier Press, Leamington Spa, England.

Process for the recovery of cyclohexanone oxime

The invention relates to a process for the recovery of cyclohexanone oxime from a solution of the oxime in toluene obtained in the preparation of cyclohexanone oxime from cyclohexanone.

According to British Patent Specification 1,303,739, such a solution can be obtained by making a solution of cyclohexanone in toluene react with a hydroxylamine salt dissolved in an aqueous buffered reaction medium. In this known process the conversion is not always complete, so that an after-reaction at elevated pH, e.g. a pH of 4.5, may be necessary to obtain a solution of oxime in toluene that contains hardly any more cyclohexanone. The final result is a solution of cyclohexanone oxime in toluene from which the oxime can be recovered by distillation.

A method for the recovery of cyclohexanone oxime from a solution of this compound in toluene by distillation has been described in British Patent Specification 1,138,750. In this known method part of the toluene is first removed in a distillation column and, next, the remainder of the toluene is removed from the residual bottom product in a second distillation column, so that the bottom product remaining in the second distillation column no longer contains toluene and consists of virtually pure cyclohexanone oxime. The toluene thus obtained still contains some oxime and is reused as a solvent in the formation of oxime.

It has now been found that cyclohexanone oxime of sufficient purity can be economically recovered by distillation from a solution of the oxime in toluene that contains over 0.5 gram of cyclohexanone per 100 grams of oxime, as obtained in the incomplete conversion of cyclohexanone, so that an after-reaction at such an elevated pH that complete conversion is achieved can be omitted in the formation of oxime.

The process according to the invention for the recovery of cyclohexanone oxime from a solution of the oxime in toluene obtained in the preparation of cyclohexanone oxime from cyclohexanone by separating the toluene and the oxime by distillation and recovering substantially pure oxime is characterized in that the distillatory separation is started from a solution of the oxime in toluene obtained by incomplete conversion of the cyclohexanone and having a cyclohexanone content of over 0.5 gram per 100 grams of oxime, and cyclohexanone oxime with less than 0.1 gram of cyclohexanone per 100 grams of oxime is recovered.

The starting solution in the process according to the invention may contain such an amount of cyclohexanone that no after-reaction at elevated pH is required in the formation of oxime. This constitutes a considerable saving, as an increase of the pH results in the production of salt, e.g. ammonium salt if the pH is raised by means of ammonia, and this salt has to be removed.

Incomplete conversion of cyclohexanone into cyclohexanone oxime may give rise to the formation of a reaction product containing oxime with a varying cyclohexanone content. The process according the the invention can be effected economically with starting solutions containing considerably more than 0.5 gram of cyclohexanone per 100 grams of oxime. If the formation of oxime is effected, for instance, at a pH of about 1.8, the resulting solution of oxime in toluene contains about 2 grams of cyclohexanone per 100 grams of oxime. However, a cyclohexanone content of the starting solution of over 35 grams per 100 grams of oxime is less recommendable and is no longer of practical significance.

The process according to the invention can be effected in practice in various ways. The distillatory separation may, for instance, be effected by means of 3 distillation columns, in the first of which toluene is distilled off, while the bottom product of the first column is fed to the top of the second column, where the desired oxime is separated as the bottom product, the top product of the second column is fed to the third column, where a top product containing toluene and cyclohexanone is separated off and a bottom product containing oxime remains which is recycled to the second column. A very suitable embodiment is the one using two distillation columns, in which toluene is distilled off in the first column, the bottom product of the first column is fed to the second column below the top, and the desired oxime is separated as the bottom product in the second column, the top product being a mixture of cyclohexanone and toluene.

As in the process according to British Patent Specification 1,138,750, the pressures in the columns in which the distillatory separation according to the invention is effected are so chosen that the cyclohexanone oxime to be recovered is not subjected to too high temperatures.

The process according to the invention is further elucidated in the following examples.

Example I

In a distillation device comprising two columns (each equipped with a heater for the bottom liquid and a condenser), a solution of 9127 kg of cyclohexanone oxime, 187 kg of cyclohexanone and 28120 kg of toluene is fed per hour to the bottom of the first column (diameter 2.5 m, 12 theoretical trays). At a reflux ratio of 0.45 and a pressure in the column top of 0.19 bar, 25450 kg of toluene, 5 kg of cyclohexanone and 1 kg of cyclohexanone oxime are discharged from the condenser per hour. The temperature in the top of the first column is 64°C and in the bottom 100°C.

The bottom product of the first column, which consists of 9126 kg of cyclohexanone oxime, 182 kg of cyclohexanone and 2670 kg of toluene per hour, is passed to the second column (diameter 1.4 m, 6 theoretical trays) and fed in at the third theoretical tray. At a reflux ratio of 0.5 and a pressure in the top of the column of 0.075 bar, 48 kg of oxime, 177 kg of cyclohexanone and 2670 kg of toluene are discharged from the condenser per hour. The bottom product obtained per hour is 9078 kg of oxime that contains only 5 kg of cyclohexanone and less than 1 kg of toluene. The temperature in the top of the second column is 40°C and in the bottom 136°C.

### Example II

In a distillation device comprising 3 columns (each equipped with a heater for the bottom liquid and a condenser), a solution of 28944 kg of toluene, 1860 kg of cyclohexanone and 8838 kg of cyclohexanone oxime is fed per hour to the bottom of the first column (diameter 2.6 m, 12 theoretical trays). At a reflux ratio of 0.9 and a top pressure of 0.19 bar, 26559 kg of toluene, 5 kg of cyclohexanone and 1 kg of oxime are discharged from the condenser per hour. The temperature in the top of the first column is 64°C and in the bottom 100°C.

The bottom product of the first column, which consists of 8837 kg of oxime, 1855 kg of cyclohexanone and 2385 kg of toluene per hour, is fed, together with the bottom product of the third column, to the top of the second column (diameter 1.4 m, 4 theoretical trays). Per hour, 8749 kg of oxime with only 4 kg of cyclohexanone are discharged from the second column as the bottom product. The temperature in the top of the second column is 57°C and in the bottom 136°C. The top pressure in the second column is 0.075 bar.

The top product of the second column, which consists of 1900 kg of oxime, 1928 kg of cyclohexanone, and 2390 kg of toluene per hour, is fed to the second theoretical tray of the third column (diameter 1.1 m, 2 theoretical trays). At a reflux ratio of 0.5 and a pressure of 0.075 bar, the top product discharged from the third column per hour is 88 kg of oxime, 1851 kg of cyclohexanone and 2385 kg of toluene. The temperature in the top of the third column is 48°C and in the bottom 125°C. The bottom product of the third column, which consists of 1812 kg of oxime, 77 kg of cyclohexanone and 5 kg of toluene per hour, is fed, together with the bottom product of the first column, to the top of the second column.

### Example III

In a distillation device comprising 3 columns (each equipped with a heater for the bottom liquid and a condenser), a solution of 9223 kg of oxime, 1945 kg of cyclohexanone and 28637 kg of toluene is fed per hour to the top of the first column. (diameter 2.6 m, 12 theoretical trays). At a reflux ratio of 0,8, a top pressure of 0.19 bar, 25472 kg of toluene, 5 kg of cyclohexanone and less than 1 kg of oxime are discharged from the condenser per hour. The temperature in the top of the first column is 64°C and in the bottom 100°C.

The bottom product of the first column, which consists of 9223 kg of oxime, 1940 kg of cyclohexanone and 3165 kg of toluene per hour, is fed, together with the top product of the second column, to the middle of the third column (diameter 1.4 m, 2 theoretical trays). At a reflux ratio of 0.35 and a top pressure of 0.075 bar, 385 kg of oxime, 1936 kg of cyclohexanone, and 3165 kg of toluene are discharged from the condenser per hour. The temperature in the top of the third column is 50°C and in the bottom 134°C.

The bottom product of the third column, which consists of 10815 kg of oxime, 387 kg of cyclohexanone, and 51 kg of toluene per hour, is fed to the top of the second column (diameter 1.4 m, 4 theoretical trays). The top product of the second column, which consists of 1977 kg of oxime, 383 kg of cyclohexanone and 51 kg of toluene is fed to the middle of the third column. The bottom product discharged from the second column consists of 8838 kg of oxime with 4 kg of cyclohexanone and less than 1 kg of toluene. The temperature in the top of the second column is 105°C and in the bottom 136°C. The top pressure in the second column is 0.075 bar.

### Claim

Process for the recovery of cyclohexanone oxime from a solution of the oxime in toluene obtained in the preparation of cyclohexanone oxime from cyclohexanone by separating the toluene and the oxime by distillation and recovering substantially pure oxime, characterized in that the distillatory separation is started from a solution of the oxime in toluene obtained by incomplete conversion of the cyclohexanone and having a cyclohexanone content of over 0.5 gram per 100 grams of oxime, and cyclohexanone oxime with less than 0.1 gram of cyclohexanone per 100 grams of oxime is recovered.

### Revendication

Procédé d'obtention de cyclohexanonoxime à partir d'une solution de l'oxime dans du toluène obtenue lors de la préparation de cyclohexanonoxime à partir de cyclohexanone, en séparant le toluène et l'oxime par distillation et en obtenant de l'oxime à peu près pure, caractérisé en ce que pour la séparation par distillation, on part d'une solution de l'oxime dans du toluène, obtenue lors de la conversion incomplète de la cyclohexanone et ayant une teneur en cyclohexanone de plus de 0,5 g par 100 g d'oxime, alors qu'on obtient de la cyclohexanonoxime avec moins de 0,1 g de cyclohexanone par 100 g d'oxime.

## Patentanspruch

Verfahren zum Zurückgewinnen von Cyclohexanonoxim aus einer Lösung des Oxims in Toluol, erhalten in der Erzeugung von Cyclohexanonoxim aus Cyclohexanon durch Abscheiden des Toluols und des Oxims durch Destillieren und Zurückgewinnen von in der Hauptsache reinem Oxim, dadurch gekennzeichnet, dass bei der destillierten Abscheidung von einer Lösung des Oxims in Toluol ausgegangen wird, erhalten durch eine unvollständige Konversion des Cyclohexanons, mit einem Cyclohexanongehalt von mehr als 0,5 Gramm je 100 Gramm Oxim, und Cyclohexanonoxim mit weniger als 0,1 Gramm Cyclohexanon je 100 Gramm Oxim zurückgewonnen wird.